(19) **Europäisches Patentamt / European Patent Office / Office européen des brevets**

(11) **EP 3 669 898 B1**

(12) # EUROPEAN PATENT SPECIFICATION

(45) Date of publication and mention
of the grant of the patent:
**20.09.2023 Bulletin 2023/38**

(21) Application number: **19209751.7**

(22) Date of filing: **18.11.2019**

(51) International Patent Classification (IPC):
**A61L 15/20** (2006.01)   **A61L 15/28** (2006.01)
**A61L 15/40** (2006.01)   **A61L 15/46** (2006.01)
**A61L 15/44** (2006.01)

(52) Cooperative Patent Classification (CPC):
(C-Sets available)
**A61L 15/46; A61L 15/20; A61L 15/28; A61L 15/40;
A61L 15/44;** A61L 2300/232; A61L 2300/30;
A61L 2300/404; A61L 2300/418; A61L 2300/606;
A61L 2400/04; A61L 2420/02; A61L 2420/06

(Cont.)

(54) **HEMOSTATIC ANTI-INFECTION WOUND DRESSING AND PREPARATION METHOD THEREOF**

HÄMOSTATISCHER INFEKTIONSSCHUTZWUNDVERBAND UND HERSTELLUNGSVERFAHREN DAFÜR

PANSEMENT ANTI-INFECTION HÉMOSTATIQUE ET SON PROCÉDÉ DE PRÉPARATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.12.2018 CN 201811554238**

(43) Date of publication of application:
**24.06.2020 Bulletin 2020/26**

(73) Proprietors:
• **Institute of Medical Support Technology of
Academy
of System Engineering of Academy of Military
Science
Tianjin City, Tianjin 300161 (CN)**
• **Tiangong University
Tainjin 300387 (CN)**

(72) Inventors:
• **GUAN, Jing
Beijing, Beijing 100141 (CN)**
• **YANG, Jian
Beijing, Beijing 100141 (CN)**
• **JING, Miaolei
Beijing, Beijing 100141 (CN)**
• **CHU, Xuexin
Beijing, Beijing 100141 (CN)**
• **LI, Zhihong
Beijing, Beijing 100141 (CN)**

• **WU, Jimin
Beijing, Beijing 100141 (CN)**
• **HUANG, Shujie
Beijing, Beijing 100141 (CN)**

(74) Representative: **Isern Patentes y Marcas S.L.
Avda. Diagonal, 463 Bis, 2°
08036 Barcelona (ES)**

(56) References cited:
• **R Thenmozhi ET AL: "Optimisation of
chitosan-honey composite film for wound
dressing application", Indian Journal of Chemical
Technology, 1 July 2016 (2016-07-01), pages
279-288, XP055687841, Retrieved from the
Internet:
URL:http://nopr.niscair.res.in/handle/1234
56789/35118 [retrieved on 2020-04-21]**
• **L SASIKALA ET AL: "Manuka Honey Loaded
Chitosan Hydrogel Films for Wound Dressing
Applications", INTERNATIONAL JOURNAL OF
PHARMTECH RESEARCH, vol. 5, no. 4, 1 October
2013 (2013-10-01) , pages 1774-1785,
XP055366819, IN ISSN: 0974-4304**
• **XIAOYAN WANG ET AL: "Exploration of Blood
Coagulation of N -Alkyl Chitosan Nanofiber
Membrane in Vitro", BIOMACROMOLECULES,
vol. 19, no. 3, 8 January 2018 (2018-01-08), pages
731-739, XP055687552, US ISSN: 1525-7797, DOI:
10.1021/acs.biomac.7b01492**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 669 898 B1

**(Cont. next page)**

- **Xiaoyan Wang ET AL: "Supporting Information for Exploration of Blood Coagulation of N-Alkyl Chitosan 2 Nanofiber in Vitro 3 Synthesis and characterization of NACS", , 1 January 2018 (2018-01-01), XP055687943, Retrieved from the Internet: URL:https://pubs.acs.org/doi/suppl/10.1021 /acs.biomac.7b01492/suppl_file/bm7b01492_s i_001.pdf [retrieved on 2020-04-21]**

Remarks:

The file contains technical information submitted after the application was filed and not included in this specification

(52) Cooperative Patent Classification (CPC): (Cont.)

C-Sets
**A61L 15/28, C08L 5/08**

**Description**

## TECHNICAL FIELD

The present invention relates to the technical field of medical dressings, and in particular to a hemostatic anti-infection wound dressing and preparation method thereof.

## BACKGROUND

Excessive hemorrhage and bacterial infection are the major factors to cause the death of the wounded in disaster areas, nearly 50% of the affected people dies from excessive hemorrhage, and almost 70% dies from bacterial infection, of which 25% of the affected people suffers bacterial infection within 10-60 min after hemorrhage. Due to some factors, e.g., destructiveness, burstiness, bad environment and not-in-time rescue in the disaster areas, the wounded always suffer hemorrhage along with infection caused by bacterial invasion, therefore, timely and effective hemostasis and anti-infection treatment and cure measures are crucial to save the life of the wounded.

Hemostatic gauze with anti-infection efficacy can be obtained by processing substrates, such as gauze, etc. by hemostatic and antibacterial agents. Chitosan has certain bactericidal and hemostatic efficacy and is usually applied in coating on gauze; Chitosan are polymerized by glucosamine monomers (including partial acetylglucosamines), whose molecules are crosslinked by hydrogen bonds, etc.; As glucosamine carries positive amino, chitosan may enter bacterial cells via permeation, absorb anionic substances, disturb the normal physiological activity of the bacterial cells, and thus has certain antibacterial efficacy, but its antibacterial effect is not obvious to common bacterial colonies on the severely-infected wound; Chitosan has certain adhesion and aggregation effect on thrombocyte and erythrocyte, be capable of activating thrombocyte to a certain extent, therefore it has certain blood coagulation efficacy, but its hemostatic effect has limitations to the large-area bleeding trauma.

There are many kinds of common antibacterial agents in the market, of which Silver Sulfadiazine (AgSD) and Povidone iodine are widely used more. AgSD has good antibacterial action to Pseudomonas aeruginosa, but requires Ag as raw materials, therefore it is expensive and not suitable for extensive promotion, meanwhile its antibacterial spectrum is narrower, and its antibacterial effect is not obvious to staphylococcus aureus, escherichia coli and other bacterial colonies, and moreover, in the aspect of whole-body metabolism of the heavy metal Ag, it remains controversial in the academic circle. Povidone iodine is a loose compound obtained by binding an element I with a polymeric carrier, it is mainly used for the disinfection of skin fungal infection and mild burns/scalds with small area, but has weaker effect on large-area bacterial infection.

## SUMMARY

In view of this, an objective of the present invention is to provide a hemostatic anti-infection wound dressing and preparation method thereof. The hemostatic anti-infection wound dressing provided by the present invention has rapid hemostasis, wide anti-infection spectrum, high metabolic rate, good biocompatibility and stability.

In order to achieve the foregoing invention objective, the present invention provides the following technical solutions:

A preparation method of a hemostatic anti-infection wound dressing, including the following steps of:

(1) dissolving N-hexylated chitosan in an acetum to obtain an N-hexylated chitosan acetum; where the degree of substitution of the N-hexylated chitosan is 8%-39%;
(2) mixing the N-hexylated chitosan acetum with glycerol and Manuka honey to obtain a mixture;
(3) coating the mixture onto a dressing substrate and then drying in order to obtain a hemostatic anti-infection wound dressing.

Preferably, the preparation method of the N-hexylated chitosan includes the following steps of:

dissolving chitosan into an acetum to obtain a chitosan acetum;
mixing the chitosan acetum with absolute ethyl alcohol and hexaldehyde to obtain a Schiff base reaction fluid; where the molar ratio of the chitosan monomeric unit to the hexaldehyde is 1:0.2-0.9;
mixing the Schiff base reaction fluid with a reducing agent for reduction reaction to obtain N-hexylated chitosan.

Preferably, the molecular weight of the chitosan is 660,000.

Preferably, the pH of the Schiff base reaction is 5-5.5, reaction time is 3-6 h and the temperature is room temperature.

Preferably, the reducing agent is one or more of sodium borohydride, potassium borohydride and lithium aluminum hydride; and
the mass ratio of the reducing agent to the chitosan monomeric unit is 2.5-3.5:1.

Preferably, the time of the reduction reaction is 12-24 h, the temperature is room temperature.

Preferably, in the mixture, the mass concentration of N-hexylated chitosan is 1-2%, the volume concentration of glycerol is 1-2% and the mass concentration of Manuka honey is 10-20%.

Preferably, the coating weight of the mixture on the dressing substrate is 300-350 mL/m$^2$.

Preferably, the drying temperature is 35-50°C, and the time is 3-5 h.

The present invention provides a hemostatic anti-infection wound dressing prepared by the method described in the above solution.

The present invention provides a preparation method for a hemostatic anti-infection wound dressing, including the following steps of: (1) dissolving N-hexylated chitosan in an acetum to obtain a N-hexylated chitosan acetum; where the degree of substitution of the N-hexylated chitosan is 8%-39%; (2) mixing the N-hexylated chitosan acetum with glycerol and Manuka honey to obtain a mixture; (3) coating the mixture onto a dressing substrate and then drying in order to obtain a hemostatic anti-infection wound dressing. N-hexylated chitosan with 8%-39% degree of substitution has significant blood coagulation and rapid hemostasis rate and has been used as a hemostatic material in the present invention; Manuka honey has strong antibacterial efficacy and stable activity, and moreover, can hardly be decomposed at room temperature and by bioactive enzymes, and is used as an antibacterial agent in the present invention; the wound dressing obtained by combining N-hexylated chitosan with Manuka honey in the present invention may rapidly and effectively achieve hemostasis, meanwhile has effects of inhibiting bacterial reproduction and preventing wound infection.

The present invention provides a hemostatic anti-infection wound dressing prepared by the above preparation method. The hemostatic anti-infection wound dressing provided by the present invention has rapid hemostasis rate and good antibacterial effect. Results of embodiments indicate that the hemostatic anti-infection wound dressing provided by the present invention has good inhibiting effect to common bacteria on a wound, moreover, the in vitro coagulation experiment shows that after applying the wound dressing of the present invention, blood coagulation time is only 142s around.

## BRIEF DESCRIPTION OF THE DRAWINGS

FIG. 1 is an infrared spectrogram of chitosan raw material and N-hexylated chitosan prepared by embodiments 1-3 of the present invention;
FIG. 2 is a diagram showing results of an in vitro coagulation experiment of blank control, chitosan raw material and N-hexylated chitosan prepared by embodiments 1-3 of the present invention;
FIG. 3 is an antibacterial observation result of a wound dressing prepared by comparative sample 1 and embodiments 4-6 of the present invention.

## DETAILED DESCRIPTION

The present invention provides a preparation method of a hemostatic anti-infection wound dressing, including the following steps of:

(1) dissolving N-hexylated chitosan in an acetum to obtain an N-hexylated chitosan acetum; where the degree of substitution of the N-hexylated chitosan is 8%-39%;
(2) mixing the N-hexylated chitosan acetum with glycerol and Manuka honey to obtain a mixture;
(3) coating the mixture onto a dressing substrate and then drying in order to obtain a hemostatic anti-infection wound dressing.

In the present invention, N-hexylated chitosan is dissolved into an acetum to obtain an N-hexylated chitosan acetum. In the present invention, the degree of substitution of the N-hexylated chitosan is 8%-39%, preferably, 8-20%, more preferably 19%.

In the present invention, the preparation method of the N-hexylated chitosan preferably includes the following steps of:

dissolving chitosan into an acetum to obtain a chitosan acetum;
mixing the chitosan acetum with absolute ethyl alcohol and hexaldehyde to obtain a Schiff base reaction fluid; where the molar ratio of the chitosan monomeric unit to the hexaldehyde is 1:0.2-0.9;

mixing the Schiff base reaction fluid with a reducing agent for reduction reaction to obtain N-hexylated chitosan.

In the present invention, chitosan is dissolved into an acetum to obtain a chitosan acetum. In the present invention, the molecular weight of the chitosan is preferably 660,000; the mass concentration of the acetum is preferably 1.5-2.5%, more preferably 2%; the ratio between the mass of the chitosan and the volume of the acetum is preferably 2 g: 100 mL; preferably, in the present invention, chitosan is added to an acetum for stirring to fully dissolve the chitosan.

In the present invention, after obtaining the chitosan acetum, the chitosan acetum is mixed with absolute ethyl alcohol and hexaldehyde for Schiff base reaction to obtain a Schiff base reaction fluid. In the present invention, the molar ratio of the chitosan monomeric unit to the hexaldehyde is preferably 1:0.2-0.9, more preferably 1:0.2-0.5, most preferably 1:0.49; in the present invention, the degree of substitution of N-hexylated chitosan may be controlled within 8-39% by controlling the molar ratio of the chitosan monomeric unit to hexaldehyde, thus improving the hemostatic effect of N-hexylated chitosan.

In the present invention, the volume ratio of the chitosan acetum to absolute ethyl alcohol is preferably 200: 75.

In the present invention, the pH of the Schiff base reaction is preferably 5-5.5, more preferably 5.1; the time of the Schiff base reaction is preferably 3-6 h, more preferably 3.5-5 h, and further preferably 4 h; the temperature of the Schiff base reaction is preferably room temperature. In embodiments of the present invention, preferably, absolute ethyl alcohol and hexaldehyde are respectively added to the chitosan acetum, and then the pH of a mixed system is regulated to 5-5.5 for Schiff base reaction; in the present invention, the Schiff base reaction time is counted when the pH is regulated to the required value. In the present invention, the pH of the mixed system is preferably regulated by hydrochloric acid, and there is no special requirement to the concentration of the hydrochloric acid as long as the pH of the mixed system is regulated to 5-5.5 in the present invention available.

At the end of the Schiff base reaction, the Schiff base reaction fluid is mixed with a reducing agent for reduction reaction to obtain N-hexylated chitosan in the present invention. In the present invention, the reducing agent is one or more of sodium borohydride, potassium borohydride and lithium aluminum hydride; the mass ratio of the reducing agent to the chitosan monomeric unit is preferably 2.5-3.5:1, more preferably 3:1.

In the present invention, the time of the reduction reaction is preferably 12-24 h, more preferably 12-13 h, and the temperature of the reduction reaction is preferably room temperature.

At the end of the reduction reaction, a reduction reaction fluid is preferably post-processed in the present invention to obtain N-hexylated chitosan. In the present invention, the post-processing preferably includes the following steps:

the pH of the reduction reaction fluid is regulated to 9.5-10.5, so that N-hexylated chitosan is separated out to obtain a solid-liquid mixture;
the solid-liquid mixture is filtered to obtain a precipitate;
the precipitate is successively washed and dried to obtain N-hexylated chitosan.

In the post-processing, the regulating agent for regulating the pH of reduction reaction fluid is preferably a sodium hydroxide solution; the mass concentration of the sodium hydroxide solution is preferably 10%; and in the present invention, the pH of the reduction reaction fluid is preferably regulated to 9.5-10.5, more preferably 10.

In the post-processing, the washing is preferably cross-washing by an ethanol solution and water; in embodiments of the present invention, it is successively washed by ethanol solution with the mass concentration of 70%, 80%, 100% and water alternatively, specifically, the precipitate is washed according to the sequence of 70% ethanol solution, water, 80% ethanol solution, water, 100% ethanol solution and water; there is no special requirement to the frequency of the washing as long as the precipitate becomes neutral in the present invention available. In the present invention, the washing water is preferably distilled water.

In the post-processing, the drying is preferably freeze drying, the freeze drying temperature is preferably -70 to -90°C, more preferably -80°C; the freeze drying pressure is preferably 15-25Pa, more preferably 20Pa; the freeze drying time is preferably 20-30 h, more preferably 24 h; in the present invention, the washed product is pre-frozen at -20°C, then frozen drying; the pre-freezing time is preferably 24 h.

In the present invention, hexyl groups are grafted on chitosan macromolecules, and the grafting number of the hexyl groups is controlled to make the degree of substitution of N-hexylated chitosan within 8-39%, which greatly improves blood coagulation property of the chitosan material.

In the present invention, N-hexylated chitosan is dissolved into an acetum to obtain an N-hexylated chitosan acetum after obtaining the N-hexylated chitosan. In the present invention, the mass concentration of the acetum is preferably 0.5-1.5%, more preferably 1%; the mass concentration of N-hexylated chitosan in the N-hexylated chitosan acetum is preferably 1.5-4%, more preferably 3.5%.

In the present invention, the pH of the N-hexylated chitosan acetum is preferably regulated to 6-6.5, more preferably 6, and then, glycerol and Manuka honey are added; in the present invention, $NaHCO_3$ aqueous solution is

preferably used to regulate the pH of N-hexylated chitosan acetum; there is no special requirement to the concentration of the NaHCO$_3$ aqueous solution as long as the pH of N-hexylated chitosan acetum is regulated to the required value in the present invention.

In the present invention, after obtaining the N-hexylated chitosan acetum, the N-hexylated chitosan acetum is mixed with glycerol and Manuka honey to obtain a mixture. In the present invention, the Manuka honey contains a Unique Manuka factor (UMF), which is a kind of special antibacterial active material with certain structure similar to phenol, thus having strong antibacterial efficacy and stable activity, and being not easy to be decomposed by temperature and enzymes. In the present invention, Manuka honey is served as an antibacterial agent so that the wound dressing has good antibacterial effect, high metabolic rate, good biocompatibility, and moreover, is not easy to produce drug-resistance bacteria.

In the present invention, the UMF of the Manuka honey is preferably 10+-20+, more preferably 20+.

In the mixture, the mass concentration of N-hexylated chitosan in the mixture is preferably 1-2%, more preferably 1.5%; the volume concentration of glycerol is preferably 1-2%, more preferably 1.5%, the mass concentration of Manuka honey is preferably 10-20%, more preferably 15-20% and further preferably 20%.

In the present invention, preferably, glycerol is added to N-hexylated chitosan acetum firstly, then Manuka honey is added. In embodiments of the present invention, preferably, firstly glycerol is added to water to form a glycerol solution, then the glycerol solution is added to N-hexylated chitosan acetum to ensure homogeneous dispersion of glycerol; the volume concentration of the glycerol solution is preferably 15-25%, more preferably 20%. In the present invention, preferably, the glycerol solution and N-hexylated chitosan acetum are mixed well, then the mixed system of glycerol and N-hexylated chitosan acetum is kept for standing to remove bubbles in the system; the standing time is preferably 30-90 min, more preferably 60 min.

In the present invention, glycerol is added to make the wound dressing obtained by coating the mixture onto substrate much softer, thus enhancing compactness and toughness of the wound dressing.

In the present invention, after obtaining the mixture, the mixture is coated on a dressing substrate to obtain the hemostatic anti-infection wound dressing after drying. In the present invention, the dressing substrate is preferably medical gauze, and the coating weight of the mixture on the dressing substrate is preferably 300-350 mL/m$^2$, more preferably 312 mL/m$^2$. In the present invention, the dressing substrate is preferably cut, then the mixture is evenly coated on the dressing substrate.

At the end of coating, the dressing substrate coated with the mixture is dried in the present invention. In the present invention, the drying temperature is preferably 35-50°C, more preferably 40°C; the drying time is preferably 3-5 h, more preferably 4 h. In the present invention, water on the surface of the dressing substrate is removed by drying to make the mixture of N-hexylated chitosan, glycerol and Manuka honey form membranoid substance loaded on the surface of the dressing substrate.

In the present invention, preferably, the obtained hemostatic anti-infection wound dressing is vacuum-sealed in the dark after drying, and sterilized by Co-60 for preservation.

The present invention provides a hemostatic anti-infection wound dressing prepared by the preparation method of the above solution, N-hexylated chitosan is used as a hemostatic material of the hemostatic anti-infection wound dressing provided by the present invention, Manuka honey is used as an antibacterial agent, therefore, the wound dressing prepared by the present invention has rapid hemostatic effect, excellent antibacterial performance and good biocompatibility, moreover, it is easy to be metabolized, not easy to produce drug-resistance bacteria, and has a broad application prospect in the field of medical dressings.

Hereby, a hemostatic anti-infection wound dressing and preparation method thereof provided by the present invention will be further described with reference to embodiments, but these embodiments should be not construed as limiting the protection scope of the present invention.

**Embodiment 1**

Preparation of N-hexylated chitosan: 2.0 g of chitosan was weighed and dissolved into 200 mL of 2% acetum for magnetic stirring for 4 h at room temperature till chitosan was fully dissolved; 75 mL of absolute ethyl alcohol was added and continuously stirred for about 1h; hexaldehyde was added according to the molar ratio of the chitosan monomeric unit to hexaldehyde being 1:0.9, then 10% HCl solution was dropwisely added to regulate pH to 5.1, above system was then continuously stirred at room temperature for 4 h to obtain a Schiff base reaction fluid;

NaBH$_4$ was added to above Schiff base reaction fluid according to the mass ratio of the chitosan monomeric unit to NaBH$_4$ as 1:3 for reduction reaction under stirring condition, and the reduction reaction time was 12 h; at the end of the reduction reaction, 10% NaOH solution was dropwisely added to the reduction reaction fluid to regulate the pH of the reaction fluid to 10, the generated precipitate products were filtered by a Buchner funnel, then washed alternatively by 70%, 80%, 100% ethanol solution and distilled water for about 3-4 times until the wash lotion became neutral.

The product N-hexylated chitosan was placed in a -20°C freezing chamber to pre-freeze for 24 h, then placed in a freeze dryer to dry for 24 h in 20Pa freeze drying condition, and after the freeze drying, N-hexylated chitosan was taken out and stored at a reaction vessel for further use.

**Embodiment 2**

Other conditions are the same as embodiment 1, except that the molar ratio of the chitosan monomeric unit to hexaldehyde was modified as 1:0.49.

**Embodiment 3**

Other conditions are the same as embodiment 1, except that the molar ratio of the chitosan monomeric unit to hexaldehyde was modified as 1:0.2.

**Infrared spectrum test:**

Fourier infrared spectrogram of chitosan and the N-hexylated chitosan obtained in embodiments 1-3 was measured by KBr pellets method, and the detailed test method was as follows: trace of sample to be tested (chitosan and N-hexylated chitosan) was taken and mixed well with chromatographic-grade KBr according to the mass ratio of 1:100, after tabletting, a FENSOR37 infrared spectrometer (FTIR) was used to acquire absorption peaks, and the data of infrared spectrogram was used to analyze whether alkyl groups were grafted onto chitosan molecules.

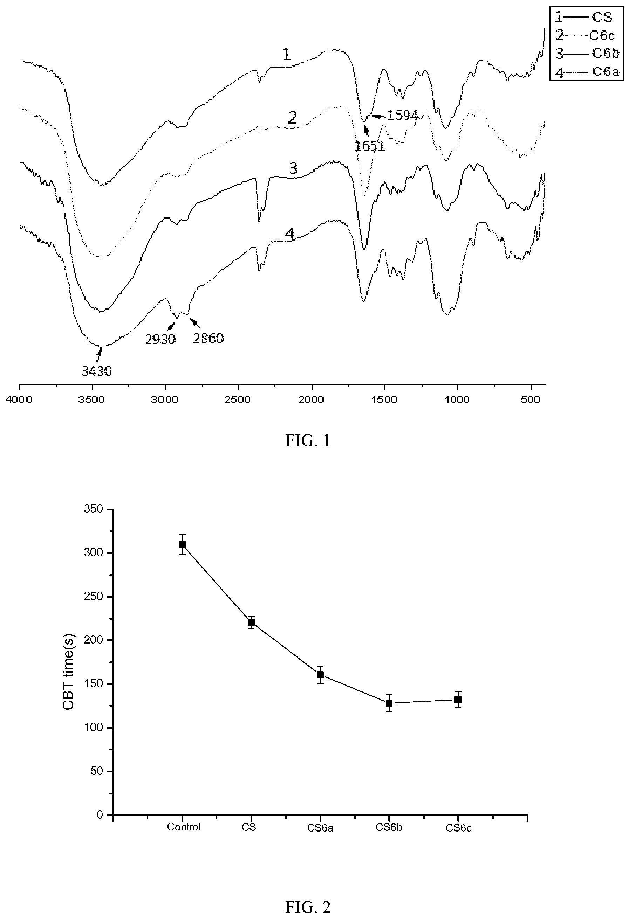

The obtained infrared spectrogram is shown in FIG. 1, in the figure, CS represents chitosan; CS6a, CS6b and CS6c respectively represent N-alkylated chitosan prepared in embodiments 1-3; according to the absorption peaks at 2930 nm and 2860 nm in FIG. 1, it can be judged that alkyl groups have been grafted onto chitosan molecules when different molar ratios of chitosan monomeric unit and hexaldehyde reacted.

**Test on the degree of substitution:**

An Elementar Vario EL cube elemental analyzer is used to measure the degree of alkyl substitution of N-alkylated chitosan prepared by embodiments 1-3, and then the degree of alkyl substitution is calculated according to the formula as shown in formula 1:

$$\frac{[8(1-x)+6x+n \times DS] \times 12}{14} = \frac{C}{N} \qquad \text{Formula 1;}$$

in the formula: x is the degree of deacetylation of chitosan;

n is the number of C in the grafted alkyl;
C/N is a mass ratio of carbon-nitrogen percentage;
DS is the degree of substitution.

Measured results were shown in table 1.

Table 1. Degree of substitution of N-alkylated chitosan prepared by embodiments 1-3

| Item | Molar ratio Chitosan monomeric unit: hexaldehyde | C(%) | N(%) | DS(%) |
|---|---|---|---|---|
| Embodiment 1 | 1:0.9 | 47.47 ± 0.89 | 6.54 ± 0.18 | 38.64 ± 0.01 |
| Embodiment 2 | 1:0.49 | 43.88 ± 2 | 6.98 ± 0.25 | 19.72 ± 1.86 |
| Embodiment 3 | 1:0.2 | 42.95 ± 0.62 | 7.50 ± 0.18 | 8.99 ± 1.32 |

**In vitro coagulation experiment:**

In vitro coagulation test used to verify the coagulation performances of chitosan and N-hexylated chitosan prepared by embodiments 1-3 includes the following steps:
20 mg of samples to be tested were weighed and put into a test tube, the tube was then placed in a 37°C water bath to pre-warm for 3 min, later on 1 mL of whole blood was added to the tube and kept for 1 min at the 37°C water bath, then

40 μL of 0.2M CaCl$_2$ solution was added as well, subsequently the test tube was taken out and inclined for observing whether blood flowed every 10 s till the blood was in semisolid state and moreover, the blood did not flow at all after the test tube was overturned 180°. Under that circumstance, blood coagulation could be judged and the corresponding time was recorded as blood coagulation time.

The obtained results are shown in FIG. 2; in the horizontal axis of FIG. 2, CS represents chitosan; CS6a, CS6b and CS6c respectively represent the N-hexylated chitosan prepared by embodiments 1-3. It can be seen from FIG. 2 that blood coagulation time shortens obviously after the N-hexylated chitosan prepared by embodiments 1-3 is added, indicating that N-hexylated chitosan prepared by the present invention may significantly improve blood coagulation efficacy after hexyl groups are grafted on chitosan; moreover, the N-hexylated chitosan prepared by embodiment 2 (the molar ratio of the chitosan monomeric unit to hexaldehyde being 1:0.49) has the best coagulation property.

**Embodiment 4**

The N-hexylated chitosan prepared by embodiment 2 was selected as a hemostatic material, 1.50 g of above N-hexylated chitosan was taken and dissolved into 100 mL of 1% acetic acid to form 1.5% N-hexylated chitosan acetum after magnetic stirring for about 4 h at room temperature to ensure that the material was fully dissolved, then 10% NaHCO$_3$ was added dropwisely into above solution until the pH of N-alkylated chitosan acetum was regulated to 6, subsequently glycerol solution composed of 2 g of glycerol and 10 mL of water was poured into above system, and stirred for 5-10 min to make the solution mix well, then the system was kept standing for 60 min to remove bubbles, finally Manuka UMF20+ honey was added to above solution, the mass concentration of the Manuka honey in the mixture was controlled to 5%; and the system was stirred for 15 min to mix Manuka honey well.

10 cm×10 cm medical gauze was cut as the dressing substrate, then 3.12 mL of above mixture was taken and coated onto the substrate to make the ratio of the mixture volume to the substrate area as 312 ml/m$^2$, then the prepared dressing was molded, dried in an oven at 40°C for 4 h, vacuum-sealed in the dark, sterilized by Co-60 and then preserved for further use.

**Embodiment 5**

Other conditions are the same as embodiment 4, except that the mass concentration of Manuka honey in the mixture was modified as 10%.

**Embodiment 6**

Other conditions are the same as embodiment 4, except that the mass concentration of Manuka honey in the mixture was modified as 20%.

**Comparative sample 1**

Other conditions are the same as embodiment 4, except that Manuka honey was not added to the mixture only.

**Evaluation of the antibacterial performance:**

The antibacterial efficacy of the wound dressing obtained from embodiments 4-6 on common bacteria was evaluated with reference to Part 1 of GB/T 20944.1-2007 Evaluation of Textiles Antibacterial Performance: Agar Plate Diffusion Method.

Staphylococcus aureus ATCC 6538, escherichia coli 8099 and pseudomonas aeruginosa CMCC(B) 10010 with the concentration of 1×10$^6$ cfu/mL were mixed well according to the ratio of 1:1:1 and selected as bacteria to be tested in future.

The wound dressing prepared by the comparative sample 1 was used as a blank control, then the wound dressings prepared by embodiments 4-6 and the comparative sample 1 were cut into circles (diameter: 21 mm±1) for further use. A nutrient agar medium was prepared in a super clean bench, and 50 μL of mixed bacteria solution was taken and evenly coated on the surface of the nutrient agar medium, the reserved wound dressing was pasted on the center of the nutrient agar medium, subsequently the agar plate was placed inversely and cultured in a bacteriological incubator for 24 h at 37°C±0.5, then the plate was taken out and the wound dressing was removed to observe whether there is a bacterial colony grown on the covering surface of the sample and as well there is an inhibition zone.

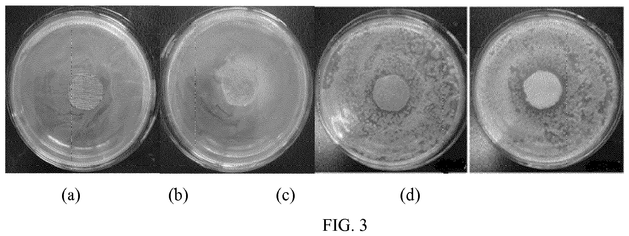

Results are shown in FIG. 3 and table 2; in FIG. 3, (a) shows-the antibacterial effect of the wound dressing prepared by the comparative sample 1; (b) shows the antibacterial effect of the wound dressing prepared by embodiment 4; (c) shows the antibacterial effect of the wound dressing prepared by embodiment 5; (d) shows the antibacterial effect of the wound dressing prepared by embodiment 6.

Table 2 In-vitro antimicrobial results of the wound dressings prepared by comparative sample 1 and embodiments 4-6

| Sample | Inhibition zone (mm) | Bacterial colony on the covering surface |
|---|---|---|
| Comparative sample 1 | 0 | Bacterial colony |
| Embodiment 4 | 0 | No bacterial colony |
| Embodiment 5 | 0.13 | No bacterial colony |
| Embodiment 6 | 0.34 | No bacterial colony |

It can be seen from FIG.3 and table 2 that the wound dressing prepared by the present invention has good inhibiting effects to common bacteria on wounds.

**In vitro coagulation experiment:**

In vitro coagulation test was used to verify the coagulation performances of the wound dressing prepared by embodiments 4-6, including the following steps:
20 mg of samples to be tested were cut into pieces and put into a test tube, the tube was then placed in a 37°C water bath to pre-warm for 3 min, later on 1 mL of whole blood was added to the tube and kept for 1 min at the 37°C water bath, and then 40 μL of 0.2M CaCl$_2$ solution was added, subsequently the test tube was taken out and inclined for observing whether blood flowed every 10 s till the blood was in semisolid state and moreover, the blood did not flow at all after the test tube was overturned 180°. Under that circumstances, blood coagulation could be judged and the corresponding time was recorded as blood coagulation time.
Experimental results show that blood coagulation time of the wound dressing was 142 s±3.6, indicating that the wound dressing provided by the present invention has good and rapid hemostatic efficacy.
The foregoing descriptions are only preferred implementation manners of the present invention. It should be noted that for a person of ordinary skill in the art, several improvements and modifications may further be made without departing from the principle of the present invention, which is defined by the appended claims.

**Claims**

**1.** A preparation method of a hemostatic anti-infection wound dressing, comprising the following steps of:

(1) dissolving N-hexylated chitosan in an acetum to obtain an N-hexylated chitosan acetum; wherein the degree of substitution of the N-hexylated chitosan is 8%-39%;
(2) mixing the N-hexylated chitosan acetum with glycerol and Manuka honey to obtain a mixture;
(3) coating the mixture onto a dressing substrate and then drying in order to obtain a hemostatic anti-infection wound dressing.

**2.** The preparation method according to claim 1, wherein the preparation method of the N-hexylated chitosan comprises the following steps of:

dissolving chitosan into an acetum to obtain a chitosan acetum;
mixing the chitosan acetum with absolute ethyl alcohol and hexaldehyde to obtain a Schiff base reaction fluid; wherein the molar ratio of the chitosan monomeric unit to the hexaldehyde is 1:0.2-0.9;
mixing the Schiff base reaction fluid with a reducing agent for reduction reaction to obtain N-hexylated chitosan.

**3.** The preparation method according to claim 2, wherein the molecular weight of the chitosan is 660,000.

**4.** The preparation method according to claim 2, wherein the pH of the Schiff base reaction is 5-5.5, the time is 3-6 h and the temperature is room temperature.

**5.** The preparation method according to claim 2, wherein the reducing agent is one or more of sodium borohydride, potassium borohydride and lithium aluminum hydride;
wherein the mass ratio of the reducing agent to the chitosan monomeric unit is 2.5-3.5:1.

6. The preparation method according to claim 2, wherein the time of the reduction reaction is 12-24 h, and the temperature is room temperature.

7. The preparation method according to claim 1, wherein the mass concentration of N-hexylated chitosan is 1-2%, the volume concentration of glycerol is 1-2% and the mass concentration of Manuka honey is 10-20%.

8. The preparation method according to claim 1 or 7, wherein the coating mount of mixture on the area of substrate is 300-350 mL/m$^2$.

9. The preparation method according to claim 1, wherein the drying temperature is 35-50°C, and the time is 3-5 h.

10. A hemostatic anti-infection wound dressing prepared by the preparation method according to any one of claims 1-9.

**Patentansprüche**

1. Herstellungsverfahren für einen hämostatischen, infektionshemmenden Wundverband, umfassend die folgenden Schritte:

   (1) Auflösen von N-hexyliertem Chitosan in einem Essig, um einen N-hexylierten Chitosanessig zu erhalten; wobei der Substitutionsgrad des N-hexylierten Chitosans 8 %-39 % beträgt;
   (2) Mischen des N-hexylierten Chitosanessigs mit Glycerin und Manuka-Honig, um eine Mischung zu erhalten;
   (3) Beschichten eines Verbandsubstrats mit der Mischung und anschließendes Trocknen, um einen hämostatischen, infektionshemmenden Wundverband zu erhalten.

2. Herstellungsverfahren gemäß Anspruch 1, wobei das Herstellungsverfahren des N-hexylierten Chitosans die folgenden Schritte umfasst:

   Auflösen von Chitosan in einem Essig, um einen Chitosanessig zu erhalten;
   Mischen des Chitosanessigs mit absolutem Ethylalkohol und Hexaldehyd, um eine Schiffsche-Base-Reaktionsflüssigkeit zu erhalten; wobei das Molverhältnis der Chitosan-Monomereinheit zum Hexaldehyd 1:0,2-0,9 beträgt;
   Mischen der Schiffsche-Base-Reaktionsflüssigkeit mit einem Reduktionsmittel für eine Reduktionsreaktion, um N-hexyliertes Chitosan zu erhalten.

3. Herstellungsverfahren gemäß Anspruch 2, wobei das Molekulargewicht des Chitosans 660.000 beträgt.

4. Herstellungsverfahren gemäß Anspruch 2, wobei der pH-Wert der Schiffschen-Base-Reaktion 5-5,5 beträgt, die Zeit 3-6 Stunden beträgt und die Temperatur Raumtemperatur ist.

5. Herstellungsverfahren gemäß Anspruch 2, wobei das Reduktionsmittel eines oder mehrere von Natriumborhydrid, Kaliumborhydrid und Lithiumaluminiumhydrid ist; wobei das Massenverhältnis des Reduktionsmittels zur Chitosan-Monomereinheit 2,5-3,5:1 beträgt.

6. Herstellungsverfahren gemäß Anspruch 2, wobei die Zeit der Reduktionsreaktion 12-24 Stunden beträgt und die Temperatur Raumtemperatur ist.

7. Herstellungsverfahren gemäß Anspruch 1, wobei die Massenkonzentration von N-hexyliertem Chitosan 1-2 %, die Volumenkonzentration von Glycerin 1-2 % und die Massenkonzentration von Manuka-Honig 10-20 % beträgt.

8. Herstellungsverfahren gemäß Anspruch 1 oder 7, wobei die Beschichtungsmenge der Mischung auf der Fläche des Substrats 300-350 ml/m$^2$ beträgt.

9. Herstellungsverfahren gemäß Anspruch 1, wobei die Trocknungstemperatur 35-50 °C und die Zeit 3-5 Stunden beträgt.

10. Hämostatischer, infektionshemmender Wundverband, hergestellt nach dem Herstellungsverfahren gemäß einem der Ansprüche 1-9.

**Revendications**

1. Procédé de préparation d'un pansement pour plaie anti-infection hémostatique, comprenant les étapes suivantes consistant à :

   (1) dissoudre du chitosane N-hexylé dans un acetum pour obtenir un acetum de chitosane N-hexylé ; le degré de substitution du chitosane N-hexylé étant de 8 % à 39 % ;
   (2) mélanger l'acetum de chitosane N-hexylé avec du glycérol et du miel de Manuka pour obtenir un mélange ;
   (3) enduire le mélange sur un substrat de pansement puis sécher afin d'obtenir un pansement pour plaie anti-infection hémostatique.

2. Procédé de préparation selon la revendication 1, dans lequel le procédé de préparation du chitosane N-hexylé comprend les étapes suivantes consistant à :

   dissoudre du chitosane dans un acetum pour obtenir un acetum de chitosane ;
   mélanger l'acetum de chitosane avec de l'alcool éthylique absolu et de l'hexanal pour obtenir un fluide de réaction vers une base de Schiff ; le rapport molaire de l'unité monomère de chitosane à l'hexanal étant de 1:0,2 à 0,9 ;
   mélanger le fluide de réaction vers une base de Schiff avec un agent réducteur pour une réaction de réduction pour obtenir du chitosane N-hexylé.

3. Procédé de préparation selon la revendication 2, dans lequel le poids moléculaire du chitosane est de 660 000.

4. Procédé de préparation selon la revendication 2, dans lequel le pH de la réaction vers une base de Schiff est de 5 à 5,5, la durée est de 3 à 6 h et la température est la température ambiante.

5. Procédé de préparation selon la revendication 2, dans lequel l'agent réducteur en est un ou plusieurs parmi le borohydrure de sodium, le borohydrure de potassium et l'hydrure d'aluminium et de lithium ;
   dans lequel le rapport massique de l'agent réducteur à l'unité monomère de chitosane est de 2,5 à 3,5:1.

6. Procédé de préparation selon la revendication 2, dans lequel la durée de la réaction de réduction est de 12 à 24 h et la température est la température ambiante.

7. Procédé de préparation selon la revendication 1, dans lequel la concentration massique de chitosane N-hexylé est de 1 à 2 %, la concentration volumique de glycérol est de 1 à 2 % et la concentration massique de miel de Manuka est de 10 à 20 %.

8. Procédé de préparation selon la revendication 1 ou 7, dans lequel la quantité d'enduction du mélange sur la surface du substrat est de 300 à 350 ml/m$^2$.

9. Procédé de préparation selon la revendication 1, dans lequel la température de séchage est de 35 à 50 °C et la durée est de 3 à 5 h.

10. Pansement pour plaie anti-infection hémostatique préparé par le procédé de préparation selon l'une quelconque des revendications 1 à 9.

FIG. 1

FIG. 2

(a)           (b)           (c)           (d)

FIG. 3